# FASCICULE DE BREVET EUROPEEN

(11) **EP 1 296 596 B1**
(45) Date de publication et mention de la délivrance du brevet: **05.04.2006**
(21) Numéro de dépôt: 01949604.1
(22) Date de dépôt: 02.07.2001
(51) Int. Cl.: A61B 10/00, A61B 17/28

(54) **PINCE A USAGE MEDICAL COMPORTANT DEUX MACHOIRES ARTICULEES**
MEDIZINISCHE ZANGE MIT ZWEI SCHWENKBAREN BACKEN
CLAMP FOR MEDICAL USE COMPRISING TWO ARTICULATED JAWS

(30) Priorité: 05.07.2000 FR 0008859
(43) Date de publication de la demande: 02.04.2003
(73) Titulaire: Flipo, Bernard, 06100 Nice (FR)
(72) Inventeur: Flipo, Bernard, 06100 Nice (FR)
(74) Mandataire: Hautier, Jean-Louis
(86) Numéro de dépôt international: PCT/FR2001/002107
(87) Numéro de publication internationale: WO 2002/002014

(56) Documents cités:
- DE-U- 9 005 519
- US-A- 3 391 690
- US-A- 4 597 385
- US-A- 5 052 402

## Description

La présente invention concerne une pince à usage médical comportant deux mâchoires articulées par pivot et commandées par le praticien depuis une zone de préhension.

L'invention trouvera particulièrement son application dans l'industrie de fabrication et dans l'utilisation d'instruments médicaux particulièrement en gynécologie pour procéder à des biopsies.

Actuellement, les pinces à biopsie telles que celles appelées « pince de Douai », présentent deux mâchoires articulées. L'une des mâchoires est bordée de lames tranchantes pour sectionner les tissus. L'autre fait le contrepoint de la mâchoire tranchante, L'ensemble forme, lorsque les mâchoires sont jointives, un volume fermé de réception des tissus sectionnés.

Le plus souvent, ce type de pince est présenté sous forme de dispositif à deux branches munies d'anneaux pour la préhension par le praticien.

Par ailleurs, une partie rectiligne intermédiaire assure la transmission de la commande des mâchoires depuis les branches à anneaux par un système de tige ou de câble coulissant.

De telles pinces présentent différents inconvénients.

En premier lieu, il s'agit de pinces complexes et coûteuses notamment en ce qui concerne le mode d'actionnement.

Ces pinces sont généralement à usage multiple et nécessitent par conséquent une stérilisation.

Elles sont d'achat coûteux et représentent un investissement important pour le praticien dans la mesure où, au-delà du coût d'achat des pinces, il fait ajouter celui de l'installation de stérilisation et de sa mise en oeuvre.

Un autre inconvénient des pinces actuelles est que leur usage est parfois délicat car on a noté de fréquentes difficultés dans le positionnement de la pince sur la zone où le prélèvement est à effectuer.

En effet, on constate que la pince a tendance à dévier ou glisser sur le tissu surtout lorsque l'on est en phase d'actionnement des mâchoires pour effectuer le prélèvement.

On connaît du document DE-U-9005519 une pince munie de dents servant, non pas à positionner l'instrument sur le tissus, mais à saisir le tissus prélevé. Cette pince n'apporte donc aucune solution au problème du positionnement de l'outil.

On connaît encore du document US-A-4.597.385 une pince munie de deux dents juxtaposées à l'extrémité distale d'une mâchoire. Ces dents sont prévues pour stabiliser le tissus durant l'utilisation. Cela étant la formation de deux dents espacées sur une mâchoire bloque la possibilité pour le praticien de pivoter, même légèrement, la pince.

Par ailleurs, les dents ne font pas saillie vers l'extérieur de la pince ce qui rend inefficace le pointage.

En outre, la partie coupante est de faible dimension et aucun moyen n'est prévu pour la réception du prélèvement.

Le document US-A-3 391 690 décrit une pince comme décrit dans les préambules des revendications 1 et 2.

La présente invention permet de remédier aux inconvénients des pinces actuelles et présente pour ce faire une nouvelle pince à usage médical.

Un premier avantage de cette pince est de présenter des moyens de pointage permettant, par ancrage dans les tissus du patient, de supprimer tout risque de déviation ou de glissement.

Il s'ensuit une plus grande précision dans le prélèvement effectué ainsi qu'une plus grande sécurité.

Par ailleurs, la pince, selon l'invention, est à l'usage plus pratique que celles connues antérieurement.

Par ailleurs, la pince ici présentée peut, dans un mode particulier de réalisation, s'affranchir d'un actionnement par tige des mâchoires. De cette façon, le prix de revient de fabrication de la pince est nettement diminué et on peut envisager sa fabrication en matière plastique pour une destination jetable.

On évitera ainsi tous les inconvénients liés à l'usage multiple des pinces et notamment les coûts et temps de stérilisation.

D'autres buts et avantages apparaîtront au cours de la description qui suit qui n'est cependant donnée qu'à titre indicatif et qui n'a pas pour but de limiter l'invention.

La présente invention concerne une pince à usage médical comportant deux mâchoires articulées par pivot et commandées par le praticien depuis une zone de préhension, caractérisée par le fait que l'une des mâchoires comporte des moyens de pointage dans les tissus du patient faisant saillie vers l'extérieur de la pince, comme décrit dans les parties caractérisantes des revendications 1 et 2.

Cette pince pourra se présenter sous les modes de réalisation introduits ci-après :
- les moyens de pointage sont constitués d'une pointe localisée à l'extrémité distale de l'une des mâchoires et faisant saillie vers l'extérieur de la pince et vers l'autre mâchoire.
- les moyens de pointage sont constitués de deux pointes chacune localisée à l'extrémité distale d'une des mâchoires et faisant saillie vers l'extérieur de la pince et vers l'autre mâchoire, les deux pointes se juxtaposant lorsque les mâchoires sont jointives.
- l'une des mâchoires présente deux lames coupantes sur sa périphérie pour sectionner des tissus.
- au moins l'une des mâchoires comporte une cavité pour la réception des tissus sectionnés.
- les lames coupantes se rejoignent à l'extrémité distale de la mâchoire qui les porte, formant deux côtés d'un triangle isocèle.
- les lames ont une longueur d'environ 3 cm et que la base du triangle est d'une longueur de 2cm environ.
- la zone de préhension est formée par deux branches à anneaux reliées par une entablure réalisant le pivot des mâchoires, lesdites mâchoires étant chacune formée dans le prolongement d'une branche, du côté de l'entablure opposé à la zone de préhension.
- la partie postérieure des branches, située du côté de l'entablure opposée à la zone de préhension, est incurvée de façon convexe et est en matériau déformable élastiquement lors de l'appui des mâchoires.
- la courbure des deux parties postérieures est symétrique.
- la partie antérieure des branches, située du même côté de l'entablure que la zone de préhension, est incurvée de façon concave et est en matériau déformable élastiquement lors de l'appui des mâchoires.
- la courbure des deux parties antérieures est symétrique.
- la distance entre l'extrémité distale des mâchoires et l'entablure est de 3 à 5 cm.
- Elle comporte :
   - deux branches reliées par une entablure et présentant à leur extrémité antérieure chacune un anneau pour constituer la zone de préhension ;
   - deux branches secondaires reliées par une entablure réalisant le pivot des mâchoires, lesdites mâchoires étant chacune formée dans le prolongement de l'extrémité postérieure d'une branche secondaire ;
   - des moyens d'articulation des extrémités postérieures des branches chacune avec l'extrémité antérieure d'une des branches secondaires.
- les moyens d'articulation comprennent :
   - deux coquilles symétriques chacune solidaire de l'extrémité antérieure d'une branche secondaire et dotées chacune d'un logement creux s'étendant dans la coquille suivant un arc de cercle sensiblement perpendiculaire et centré relativement à l'axe passant par les deux entablures, lesdits logements creux étant pourvus d'une ouverture orientée à l'opposé de la branche secondaire ;
   - deux noyaux chacun solidaire de l'extrémité postérieure d'une branche et logés dans le logement creux des coquilles, les noyaux étant stoppés en translation dans leur logement creux.
- les moyens d'articulation comprennent :
   - deux coquilles symétriques chacune solidaire de l'extrémité postérieure d'une branche et dotées chacune d'un logement creux s'étendant dans la coquille suivant un arc de cercle sensiblement perpendiculaire et centré relativement à l'axe passant par les deux entablures lesdits logements creux étant pourvus d'une ouverture orientée à l'opposé de la branche ;
   - deux noyaux chacun solidaire de l'extrémité antérieure d'une branche secondaire et logés dans le logement creux des coquilles, les noyaux étant stoppés en translation dans leur logement creux.
   - les noyaux sont sphériques,
   - les logements creux comprennent des cavités de formes et dimensions complémentaires à celles des noyaux.
   - la mâchoire présentant les lames coupantes comporte deux logements dans lesquels les lames sont enchâssées.
   - la pince est en matière plastique.

Les dessins ci-joints sont donnés à titre d'exemples indicatifs et non limitatifs. Ils représentent un mode de réalisation préféré selon l'invention. Ils permettront de comprendre aisément l'invention.
La figure 1 est une vue générale de la pince selon l'invention dans un mode de réalisation préféré.
La figure 2 est une vue partielle en coupe de la mâchoire en position jointive.
La figure 3 est une vue partielle en coupe de la partie mâchoire en position écartée.
La figure 4 est une vue de dessous partielle de la mâchoire supérieure.
La figure 5 est une vue de dessus partielle de la mâchoire inférieure.
La figure 6 montre une variante de conformation des branches de la pince ; la figure 10 est une autre possibilité.
Les figures 7 à 9 montrent un mode particulier de réalisation des mâchoires. Les figures 11 et 12 en illustrent la position fermée.
Les figures 13 à 18 présentent une autre variante de l'invention avec un degré de liberté supplémentaire.

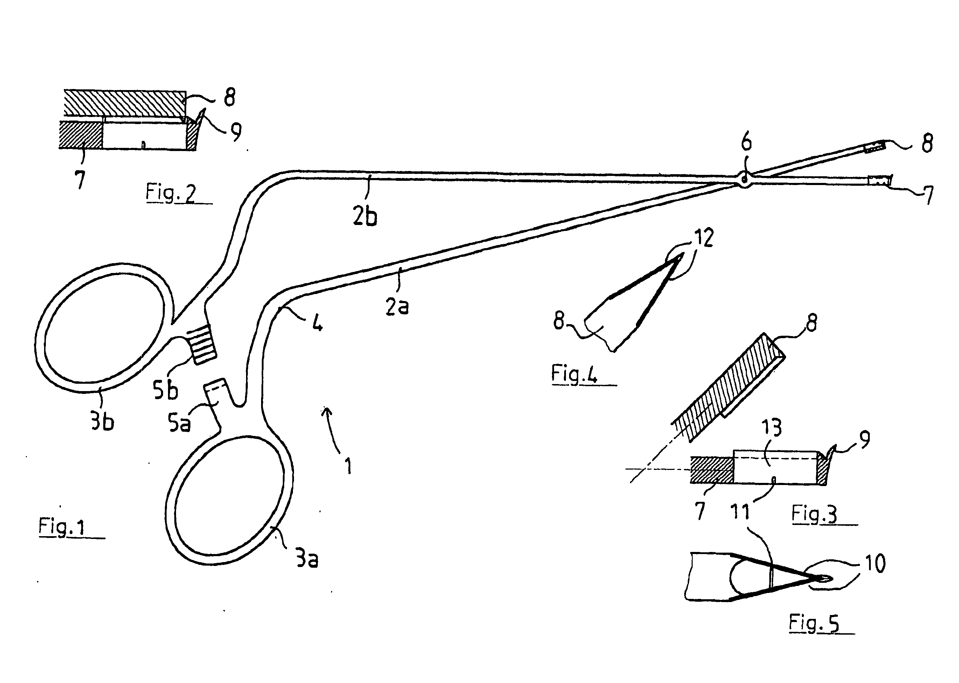

La figure 1 donne un exemple de physionomie que pourra présenter la pince selon l'invention bien que cet exemple soit indicatif et n'exclut pas d'autres configurations de réalisation de la pince.

Selon l'exemple de la figure 1, la pince comprend deux mâchoires repérées 7 et 8 articulées par un pivot de façon à pouvoir être rapprochées jusqu'à une position jointive illustrée en figure 2 ou écartée comme c'est le cas en figure 3.

Le rapprochement ou l'écartement des mâchoires 7,8 est commandé par le praticien depuis une zone de préhension 1.

Dans le cas illustré, cette zone de préhension 1 comprend deux anneaux 3a, 3b formés à l'extrémité de deux branches 2a, 2b.

Eventuellement, les branches 2a, 2b, comportent un coude 4. Par ailleurs, des pattes de fermeture 5a, 5b peuvent être constituées de manière connue afin de permettre le maintien de la pince en position fermée. L'emploi de telles pattes de fermeture est cependant accessoire.

Le cas de l'exemple de la figure 1, l'articulation par pivot existant entre les deux mâchoires 7,8 est réalisée par l'intermédiaire d'une entablure 6 comme c'est le cas pour une simple paire de ciseaux.

Dans ce cadre, les mâchoires 7 et 8 sont situées dans le prolongement des branches 2a, 2b, mais de l'autre côté de l'entablure 6 par rapport à la zone de préhension 1.

Les mâchoires 7, 8 peuvent être constituées d'un seul tenant, dans la matière des branches 2a, 2b, ou être des pièces rapportées à leur extrémité.

Selon l'invention, l'une des mâchoires 7,8 comporte des moyens de pointage dans les tissus du patient.

En effet, on a remarqué qu'un besoin important existait dans la réalisation d'un ancrage de la pince au cours du travail du praticien.

L'avantage d'une telle possibilité d'ancrage se fait notamment sentir lors de réalisation de prélèvements de tissus ou encore de biopsies.

Dans un mode préféré de réalisation parfaitement illustré aux figures 2 et 3, les moyens de pointage ont la forme d'une pointe 9 localisée à l'extrémité distale de l'une des mâchoires 7.

En se référant à la figure 3, on constate que l'orientation de la pointe 9 peut être particulière.

En effet, il est avantageux de constituer la pointe 9 orientée non seulement vers l'extérieur en faisant saillie au-delà de l'extrémité distale de la mâchoire 7 mais aussi vers l'autre mâchoire 8.

Suivant cette configuration, le praticien pourra maintenir facilement en position, la pince, en exerçant une légère pression de la pointe 9 sur le tissu et en maintenant son effort légèrement orienté vers le haut.

On notera aussi que cette configuration permet une pénétration suffisante dans le tissu sans pour autant s'engager en profondeur dans celui-ci.

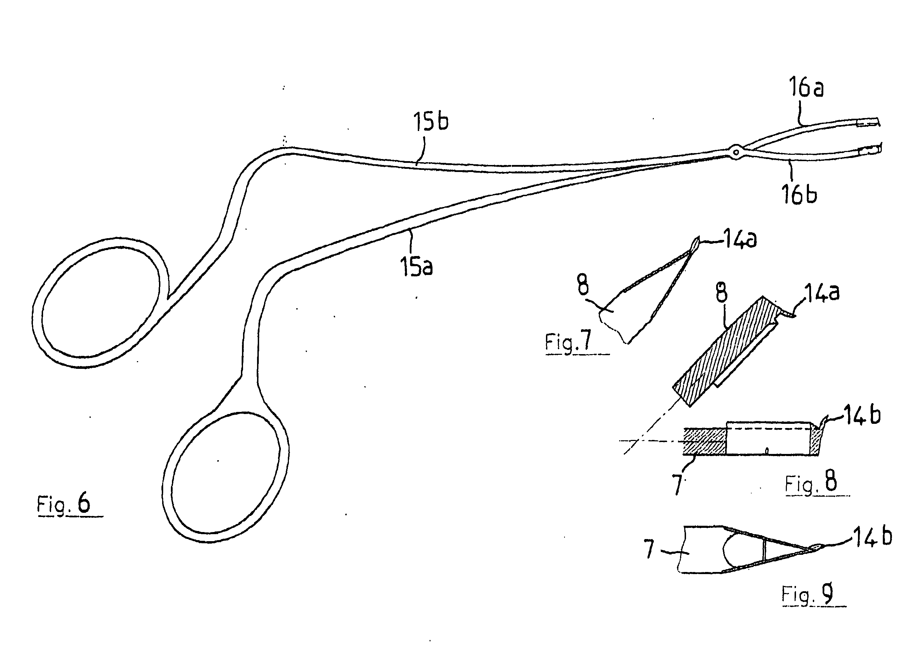

Les figures 7 à 9 et 11 et 12 montrent la formation de deux pointes 14a, 14b, chacune sur une mâchoire 7, 8. De cette façon, le praticien peut choisir la mâchoire 7, 8 qu'il souhaite pointer dans le tissus, ce qui est avantageux pour la souplesse du positionnement de la pince. Les pointes 14a, 14b sont orientées chacune vers un côté de la pince pour permettre leur juxtaposition lors de la fermeture.

La pince ici présentée s'appliquera particulièrement à la réalisation de biopsie.

Dans ce cadre, l'une des mâchoires, la mâchoire 7, présente deux lames coupantes 10 afin de sectionner les tissus.

Telles que représentées en figure 5, les lames 10, sont présentes à proximité des bords de la mâchoire 7.

Dans le mode préféré illustré à la figure 5, la mâchoire 7 ainsi que la mâchoire 8 ont une forme sensiblement triangulaire à leur extrémité.

Dans ce cadre, les lames coupantes à l'extrémité distale de la mâchoire forment ainsi un triangle isocèle.

A titre d'exemple, les lames pourront avoir une longueur de trois centimètres chacune, alors que la base du triangle ainsi formé aura une longueur de deux centimètres environ.

En se référant aux figures 3 à 5, on comprend qu'il est possible de sectionner les tissus au moyen des lames coupantes 10 par leur coopération avec les bords 12 présents de façon complémentaire sur la mâchoire 8.

Toujours dans le cadre préféré de la réalisation de biopsie, la mâchoire peut présenter une cavité 13 pour recevoir le tissu prélevé.

Comme indiqué en figure 3 et 5 à titre d'exemple, la cavité 13 peut être délimitée en partie inférieure de la mâchoire 7 par une configuration à claire-voie composée par une ou plusieurs barres 11.

La figure 2 montre que, lorsque les mâchoires 7 et 8 sont jointives, la cavité formée dans la mâchoire 7 permet de constituer un espace interstitiel sensiblement fermé.

Dans le cas de la formation d'une pince composée de deux branches 2a, 2b, reliée par une entablure 6, la distance entre l'extrémité distale 7, 8 et l'entablure pourra être de trois à cinq centimètres.

Selon une variante préférée de l'invention, la pince ici présentée est destinée à être jetable.

Dans ce cadre, il est avantageux de la constituer essentiellement en matière plastique, particulièrement en polycarbonate ou en polyamide.

Pour ce faire, la majorité des éléments constitutifs de la pince sera constituée en matériaux plastiques et les lames 10 pourront, soit être formées dans l'ensemble, soit être rapportées et formées en toute matière courante notamment métallique sous forme de lames de rasoir.

Pour la fixation et le maintien en position des lames 10 sur la mâchoire 7, cette dernière pourra comporter des logements non représentés sur les figures mais s'étendant sensiblement à l'endroit où l'on désire enchâsser les lames coupantes 10.

Les logements comporteront chacun une fente s'étendant le long des bords de la mâchoire 7 et recevant les lames 10.

On constitue ainsi une pince dont la manipulation et l'emploi sont pratiques car présentant des moyens de pointage et permettant sa réalisation à moindre coût de façon à constituer un élément jetable.

Selon une variante préférée, la pince comporte des branches incurvées pour exercer un effort supplémentaire sur les mâchoires, après leur mise en contact, par déformation élastique des branches.

Particulièrement pour une réalisation en matière plastique, il est important de préserver des caractéristiques optimales en forcipressure, lorsque les mâchoires (7, 8) sont fermées.

Pour ce faire, on propose préférentiellement de donner une forme incurvée aux branches (2a, 2b), permettant une déformation élastique des branches lorsque les mâchoires sont en contact, pour augmenter l'effort appliqué.

On utilisera un matériau dont les propriétés de déformation élastique sont en rapport avec le niveau de déformation souhaité pour les branches (2a, 2b).

La courbure donnée aux branches (2a, 2b) est inverse à celle qu'elle a tendance à adopter lorsque l'on force l'appui des mâchoires (7, 8).

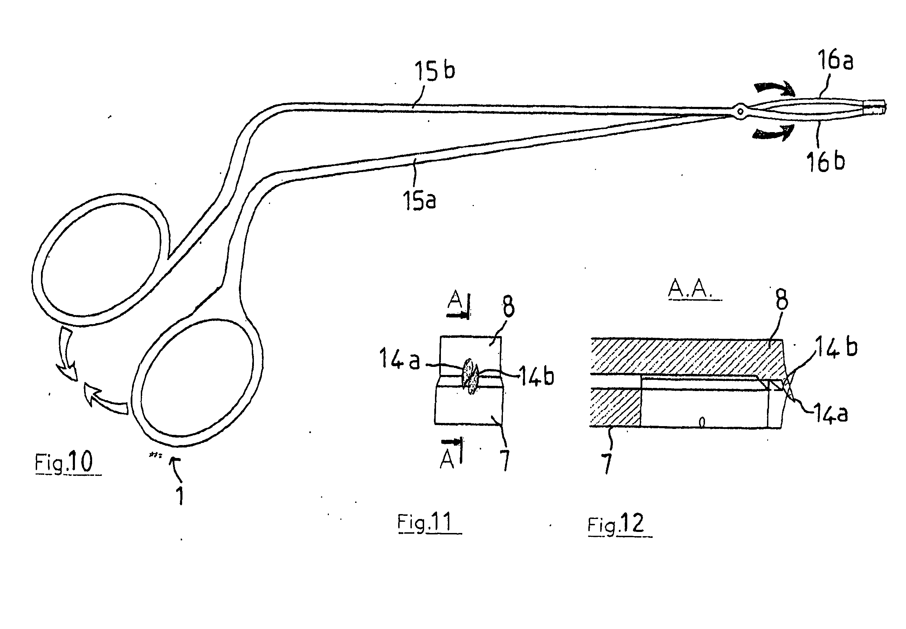

En se référant à la figure 10, on forme des parties postérieures (16a, 16b) des branches (2a, 2b) (ces parties sont celles situées du côté opposé à la zone de préhension (1)) ayant une courbure convexe, préférentiellement de façon symétrique.

Les flèches pleines en figure 10, illustrent le sens de la déformation élastique qui a tendance à annuler la courbure convexe.

De façon supplémentaire ou alternative, les parties antérieures (15a, 15b) des branches (2a, 2b) (ces parties sont du même côté que la zone de préhension (1)) ont une courbure concave, comme le montre la figure 6.

La réalisation d'une telle pince à branches courbées telles qu'en figure 6 et 10 est possible par ailleurs avec tout type et conception de mâchoires.

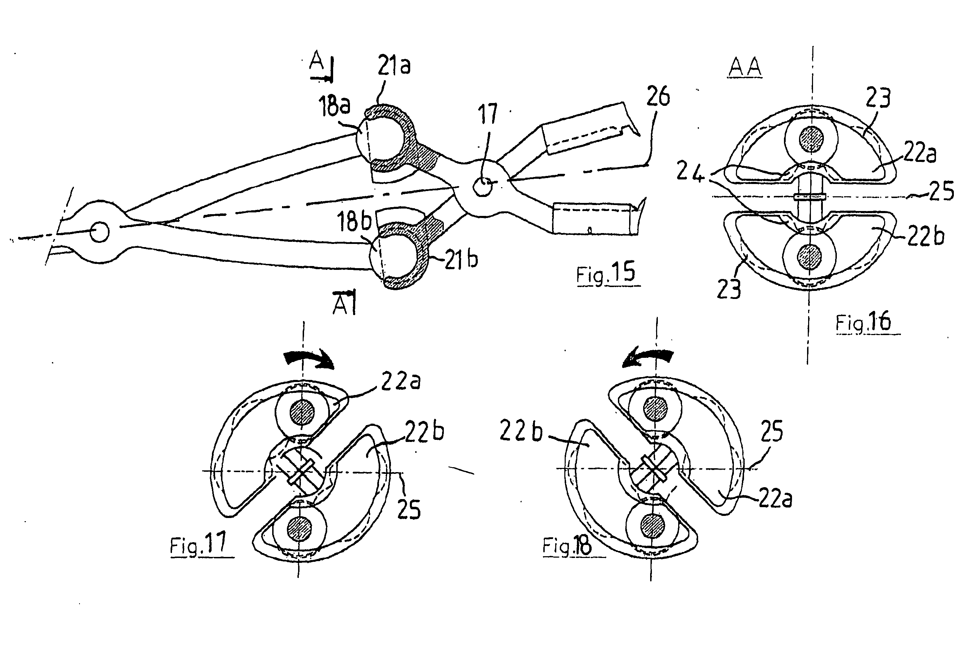

On décrit ci-après une autre variante de l'invention en référence aux figures 14 à 18.

Selon ce mode de réalisation, il est possible d'opérer un pivot relatif entre la partie de la pince comportant les mâchoires et la partie de la pince comportant la zone de préhension 1.

Cette liberté en rotation additionnelle permet d'ajuster au mieux l'orientation des mâchoires lorsqu'un prélèvement de tissus ou tout autre opération est à réaliser.

On notera que la réalisation de ce degré de liberté supplémentaire est utilisable pour une pince avec tout type de mâchoires.

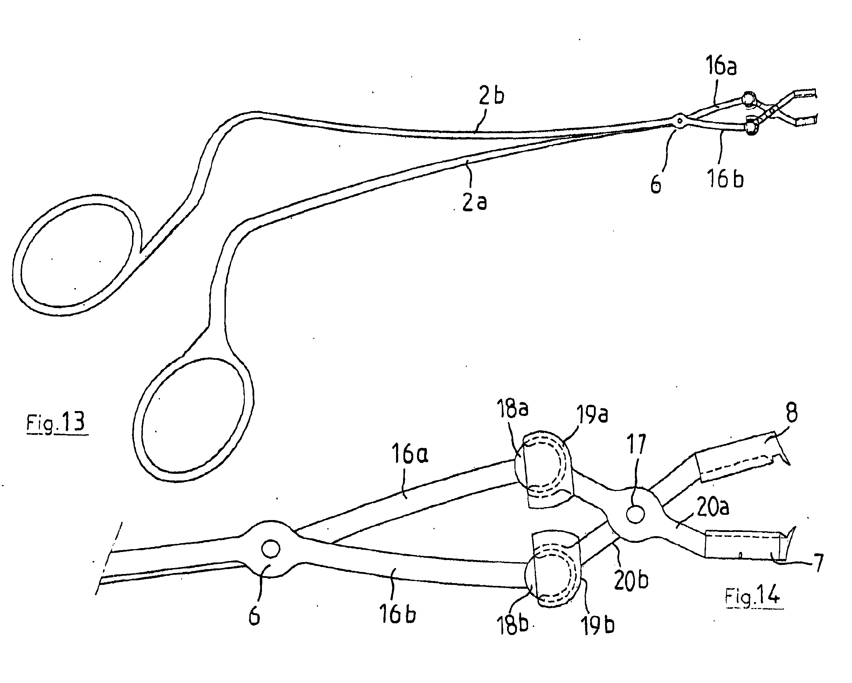

En référence aux figures 13 et 14, on forme une zone de préhension ainsi que des branches 2a, 2b sensiblement équivalentes à celles décrites dans le mode de réalisation précédent.

Cela étant, les parties postérieures 16a, 16b des branches 2a, 2b ne portent plus ici les mâchoires 7, 8.

Les mâchoires 7, 8 sont en effet portées par des branches secondaires 20a, 20b reliées par pivot par une entablure 17.

Le degré de rotation supplémentaire est rendu possible au niveau de la liaison entre les branches 2a, 2b et les branches secondaires 20a, 20b.

Cette liaison s'effectue plus précisément entre les parties postérieures 16a et 16b des branches 2a, 2b et les parties antérieures des branches secondaires 20a, 20b.

Il apparaît immédiatement que l'ouverture et la fermeture des mâchoires 7, 8 est opérée de façon courante au niveau des anneaux 3a, 3b de la zone de préhension mais par l'intermédiaire de deux rotations au niveau des entablures 6 et 17.

L'articulation supplémentaire conférée entre les branches 2a, 2b et 20a, 20b assurent un réglage de l'orientation des mâchoires 7, 8.

Selon le mode de réalisation présentée aux figures, ces moyens d'articulation sont sensiblement symétriques pour respectivement les branches 2a, 20a et 2b, 20b.

Pour chacune de ces paires de branches, on forme une coquille 19a, 19b qui, dans le cas illustré aux figures est solidaire de l'extrémité antérieure des branches secondaires 20a, 20b.

Les coquilles 19a, 19b comportent une ouverture orientée à l'opposé des branches 20a, 20b et donnant accès à un logement creux 22a, 22b formé dans le volume intérieur des coquilles 19a, 19b.

On a illustré plus particulièrement aux figures 16 à 18 la forme conférée aux coquilles 19a, 19b.

En effet, pour réaliser le mouvement de rotation supplémentaire, le logement creux 22a, 22b a une forme sensiblement en arc de cercle disposé dans un plan perpendiculaire à l'axe 26 commun aux deux entablures 6 et 17, cet arc de cercle étant en outre centré sur cet axe 26.

On note également aux figures que la conformation des coquilles 19a, 19b et particulièrement de leur logement creux 22a, 22b est symétrique.

Chaque logement 22a, 22b reçoit un noyau 18a, 18b solidaire de l'extrémité correspondante d'une branche 2a, 2b.

Le noyau 18a, 18b est mobile en rotation dans le logement 22a, 22b mais est stoppé en translation et maintenu à l'intérieur du logement creux 22a par tout moyen courant ou préférentiellement par le biais d'une dimension adaptée de l'ouverture des logements 22a, 22b suffisamment inférieure à la dimension des noyaux 18a, 18b pour les retenir.

On comprend aisément qu'il est possible d'inverser la disposition des coquilles 19a, 19b et des noyaux 18a, 18b, et que par conséquent les coquilles 19a, 19b peuvent être réalisées à l'extrémité postérieure des branches 2a, 2b et que les noyaux 18a, 18b peuvent être réalisés à l'extrémité antérieure des branches secondaires 20a, 20b.

Toujours en référence aux figures 13 à 18, les noyaux 18a, 18b sont sensiblement sphériques et coopèrent avec des cavités 23, 24 formées à l'intérieur des logements creux 22a, 22b.

De cette façon, les noyaux 18a, 18b ont une propension à se loger dans l'une des cavités 23, 24 et ne sont déplaçables qu'avec un léger effort supplémentaire le long de l'arc de cercle défini par les logements creux 22a, 22b.

On forme ainsi, suivant le nombre et la disposition des cavités 23, 24, une pluralité de positions d'arrêt des noyaux 18a, 18b dans les coquilles 19a, 19b.

Le praticien a donc le choix entre une multiplicité de positions relatives de la zone de préhension 1 et des mâchoires 7, 8.

Il est enfin précisé que pour l'ensemble de la description exposée ci-dessus, on appelle antérieure toute partie d'un organe de la pince selon l'invention disposée vers l'arrière de la pince c'est à dire vers la zone de préhension 1.

A l'opposé, on appelle postérieure toute partie d'une organe constitutif de la pince disposée vers l'avant de la pince c'est-à-dire vers l'emplacement des mâchoires 7, 8.

### REFERENCES

1. Zone de préhension
2a, 2b. Branches
3a, 3b. Anneaux
4. Coude
5a, 5b. Pattes de fermeture
6. Entablure
7. Mâchoire inférieure
8. Mâchoire supérieure
9. Pointe
10. Lames
11. Barre
12. Bords
13. Cavité
14a, 14b. Pointes
15a, 15b. Parties antérieures
16a, 16b. Parties postérieures
17. Entablure secondaire
18a, 18b. Noyaux
19a, 19b. Coquilles
20a, 20b. Branches secondaires
21a, 21b. Enveloppe
22a, 22b. Logements creux
23. Cavités
24. Cavités
25. Axe transversal à l'entablure 6
26. Axe commun aux entablures 6, 17

## Revendications

1. Pince à usage médical comportant deux mâchoires (7,8) articulées par pivot et commandées par le praticien depuis une zone de préhension (1) dans laquelle :
- l'une (7) des mâchoires (7,8) comporte des moyens de pointage dans les tissus du patient faisant saillie vers l'extérieur de la pince quand les mâchoirs sont fermées et localisés à l'extrémité distale de l'une des mâchoires (7,8) ;
- l'une (7) des mâchoires (7,8) présente deux lames (10) coupantes sur sa périphérie pour sectionner des tissus ;
- au moins l'une des mâchoires (7,8) comporte une cavité (13) pour la réception des tissus sectionnés, **caractérisée par le fait**
**que** les moyens de pointage sont constitués d'une pointe (9) faisant saillie vers l'extérieur de la pince et vers l'autre mâchoire(8).

2. Pince à usage médical comportant deux mâchoires (7,8) articulées par pivot et commandées par le praticien depuis une zone de préhension (1) dans laquelle :
- l'une (7) des mâchoires (7,8) comporte des moyens de pointage dans les tissus du patient faisant saillie vers l'extérieur de la pince quand les mâchoirs sont fermées et localisés à l'extrémité distale de l'une des mâchoires (7,8) ;
- l'une (7) des mâchoires (7,8) présente deux lames (10) coupantes sur sa périphérie pour sectionner des tissus ;
- au moins l'une des mâchoires (7,8) comporte une cavité (13) pour la réception des tissus sectionnés, **caractérisée par le fait**
**que** les moyens de pointage sont constitués de deux pointes (14a, 14b) chacune localisée à l'extrémité distale d'une des mâchoires (7, 8) et faisant saillie vers l'extérieur de la pince et vers l'autre mâchoire, les deux pointes (14a, 14b) se juxtaposant lorsque les mâchoires (7, 8) sont jointives.

3. Pince à usage médical selon l'une quelconque des revendications 1 ou 2, **caractérisée par le fait**
**que** les lames coupantes (10) se rejoignent à l'extrémité distale de la mâchoire (7) qui les porte, formant deux côtés d'un triangle isocèle.

4. Pince à usage médical selon la revendication 3, **caractérisée par le fait**
**que** les lames (10) ont une longueur d'environ trois centimètres et que la base du triangle est d'une longueur de deux centimètres environ.

5. Pince à usage médical selon l'une quelconque des revendications 1 à 4, **caractérisée par le fait**
**que** la zone de préhension (1) est formée par deux branches (2a, 2b) à anneaux (3a, 3b) reliées par une entablure (6) réalisant le pivot des mâchoires (7,8), lesdites mâchoires (7,8) étant chacune formée dans le prolongement d'une branche (2a, 2b), du côté de l'entablure (6) opposé à la zone de préhension (1).

6. Pince multifonctions à usage médical selon la revendication 5 **caractérisée par le fait**
**que** la partie postérieure (16a, 16b) des branches (2a, 2b), située du côté de l'entablure (6) opposée à la zone de préhension (1), est incurvée de façon convexe et est en matériau déformable élastiquement lors de l'appui des mâchoires (7, 8).

7. Pince multifonctions à usage médical selon la revendication 6 **caractérisée par le fait que**
la courbure des deux parties postérieures (16a, 16b) est symétrique.

8. Pince multifonctions à usage médical selon l'une quelconque des revendications 5 à 7, **caractérisé par le fait**
**que** la partie antérieure (15a, 15b) des branches (2a, 2b), située du même côté de l'entablure (6) que la zone de préhension (1), est incurvée de façon concave et est en matériau déformable élastiquement lors de l'appui des mâchoires (7, 8).

9. Pince multifonctions à usage médical selon la revendication 8 **caractérisée par le fait**
**que** la courbure des deux parties antérieures (15a, 15b) est symétrique.

10. Pince multifonctions à usage médical selon l'une quelconque des revendications 5 à 9, **caractérisée par le fait**
**que** la distance entre l'extrémité distale des mâchoires (7,8) et l'entablure (6) est de trois à cinq centimètres.

11. Pince multifonctions à usage médical selon l'une quelconque des revendications 1 à 4 **caractérisée par le fait**
**qu'**elle comporte :
- deux branches (2a, 2b) reliées par une entablure (6) et présentant à leur extrémité antérieure chacune un anneau (3a, 3b) pour constituer la zone de préhension (1) ;
- deux branches secondaires (20a, 20b) reliées par une entablure (17) réalisant le pivot des mâchoires (7, 8), lesdites mâchoires (7, 8) étant chacune formée dans le prolongement de l'extrémité postérieure d'une branche secondaire (20a, 20b) ;
- des moyens d'articulation des extrémités postérieures des branches (2a, 2b) chacune avec l'extrémité antérieure d'une des branches secondaires (20a, 20b).

12. Pince multifonctions à usage médical selon la revendication 11 **caractérisée par le fait**
**que** les moyens d'articulation comprennent :
- deux coquilles (19a, 19b) symétriques chacune solidaire de l'extrémité antérieure d'une branche secondaire (20a, 20b) et dotées chacune d'un logement creux (22a, 22b) s'étendant dans la coquille (19a, 19b) suivant un arc de cercle sensiblement perpendiculaire et centré relativement à l'axe (26) passant par les deux entablures (6, 17), lesdits logements creux (22a, 22b) étant pourvus d'une ouverture orientée à l'opposé de la branche secondaire (20a, 20b) ;
- deux noyaux (18a, 18b) chacun solidaire de l'extrémité postérieure d'une branche (2a, 2b) et logés dans le logement creux (22a, 22b) des coquilles (19a, 19b), les noyaux (18a, 18b) étant stoppés en translation dans leur logement creux (22a, 22b).

13. Pince multifonctions à usage médical selon la revendication 11 **caractérisée par le fait**
**que** les moyens d'articulation comprennent :
- deux coquilles (19a, 19b) symétriques chacune solidaire de l'extrémité postérieure d'une branche (2a, 2b) et dotées chacune d'un logement creux (22a, 22b) s'étendant dans la coquille (19a, 19b) suivant un arc de cercle sensiblement perpendiculaire et centré relativement à l'axe (26) passant par les deux entablures (6, 17) lesdits logements creux (22a, 22b) étant pourvus d'une ouverture orientée à l'opposé de la branche (2a, 2b) ;
- deux noyaux (18a, 18b) chacun solidaire de l'extrémité antérieure d'une branche secondaire (20a, 20b) et logés dans le logement creux (22a, 22b) des coquilles (19a; 19b), les noyaux (18a, 18b) étant stoppés en translation dans leur logement creux (22a, 22b).

14. Pince multifonctions à usage médical selon la revendication 12 ou 13 **caractérisée par le fait que**
- les noyaux (18a, 18b) sont sphériques,
- les logements creux (22a, 22b) comprennent des cavités (23, 24) de formes et dimensions complémentaires à celles des noyaux (18a, 18b).

## Patentansprüche

1. Zange für medizinische Zwecke mit zwei Backen (7,8), die gelenkig miteinander verbunden sind und vom Arzt aus einer Greifzone (1) gesteuert werden, bei der:
- eine (7) der Backen (7, 8) Mittel zum Einstechen der Gewebe des Patienten besitzt, die nach außen von der Zange abstehen, wenn die Backen geschlossen sind und die am distalen Ende einer der Backen (7, 8) angeordnet sind;
- eine (7) der Backen (7, 8) auf ihrem Umfang zwei Schneiden (10) besitzt, um die Gewebe zu schneiden;
- mindestens eine der Backen (7, 8) eine Ausnehmung (13) zur Aufnahme des zerschnittenen Gewebes besitzt, **gekennzeichnet dadurch, dass** die Mittel zum Einstechen aus einer Spitze (9) bestehen, die von der Zange nach außen hervorsteht und auf die andere Backe (8) gerichtet ist.

2. Zange für medizinische Zwecke mit zwei Backen (7, 8), die gelenkig miteinander verbunden sind und vom Arzt aus einer Greifzone (1) gesteuert werden, bei der:
- eine (7) der Backen (7, 8) Mittel zum Einstechen der Gewebe des Patienten besitzt, die nach außen von der Zange abstehen, wenn die Backen geschlossen sind und die am distalen Ende einer der Backen (7, 8) angeordnet sind;
- eine (7) der Backen (7, 8) auf ihrem Umfang zwei Schneiden (10) besitzt, um die Gewebe zu schneiden;
- mindestens eine der Backen (7, 8) eine Ausnehmung (13) zur Aufnahme des zerschnittenen Gewebes besitzt, **gekennzeichnet dadurch, dass** die Mittel zum Einstechen aus zwei Spitzen (14a, 14b) bestehen, die beide von der Zange nach außen hervorstehen und auf die andere Backe (8) gerichtet sind, wobei die beiden Spitzen (14a, 14b) sich nebeneinander legen, wenn die Backen (7, 8) aneinander anliegen.

3. Zange für medizinische Zwecke gemäß einem der Ansprüche 1 bis 2, **gekennzeichnet dadurch,**
- **dass** die Schneiden (10) am Ende der sie tragenden Backe (7) aufeinander zulaufen und zwei Seiten eines gleichseitigen Dreiecks bilden.

4. Zange für medizinische Zwecke gemäß Anspruch 3, **gekennzeichnet dadurch, dass**
die Schneiden (10) eine Länge von etwa drei Zentimetern haben und die Basis des Dreiecks eine Länge von etwa zwei Zentimetern.

5. Zange für medizinische Zwecke gemäß einem der Ansprüche 1 bis 4, **gekennzeichnet dadurch,**
**dass** die Greifzone (1) aus zwei Griffen (2a, 2b) mit Ringen (3a, 3b) gebildet wird, die an einer Flachstelle (6), an der sich das Gelenk der Backen (7, 8) befindet, miteinander verbunden sind, sowie **dadurch**, dass jede der Backen (7, 8) in der Verlängerung eines Griffes (2a, 2b), auf der, in bezug auf die Flachstelle (6), der Greifzone (1) gegenüberliegenden Seite ausgebildet sind.

6. Mehrzweckzange für medizinische Zwecke gemäß Anspruch 5, **gekennzeichnet dadurch,**
**dass** der vordere Teil 16a, 16b) der Griffe (2a, 2b), der auf der, bezogen auf die Flachstelle (6), der Greifzone (1) gegenüberliegenden Seite liegt, konvex geformt ist und aus einem Werkstoff besteht, der sich beim Anlegen der Backen (7, 8) elastisch verformt.

7. Mehrzweckzange für medizinische Zwecke gemäß Anspruch 6, **gekennzeichnet dadurch,**
**dass** die Bogenformen der beiden hinteren Bereiche (16a, 16b) symmetrisch sind.

8. Mehrzweckzange für medizinische Zwecke gemäß einem der Ansprüche 5 bis 7, **gekennzeichnet dadurch, dass**
der vordere Teil (15a, 16b) der Griffe (2a, 2b), der auf der, bezogen auf die Flachstelle (6), der Greifzone (1) gleichen Seite liegt, konkav geformt ist und aus einem Werkstoff besteht, der sich beim Anlegen der Backen (7, 8) elastisch verformt.

9. Mehrzweckzange für medizinische Zwecke gemäß Anspruch 8, **gekennzeichnet dadurch, dass**
die Bogenformen der beiden vorderen Bereiche (15a, 15b) symmetrisch sind.

10. Mehrzweckzange für medizinische Zwecke gemäß einem der Ansprüche 5 bis 9, **gekennzeichnet dadurch, dass**
die Entfernung zwischen dem distalen Ende der Backen (7, 8) und der Flachstelle (6) drei bis fünf Zentimeter beträgt.

11. Zange für medizinische Zwecke gemäß einem der Ansprüche 1 bis 4, **gekennzeichnet dadurch,**
**dass** sie folgende Teile besitzt:
- zwei Griffe (2a, 2b), die an einer Flachstelle (6) miteinander verbunden sind und an ihrem vorderen Ende jeder einen Ring (3a, 3b) besitzen, um eine Greifzone (1) zu bilden;
- zwei sekundäre Griffe (20a, 20b), die an einer Flachstelle (17) miteinander verbunden sind und das Gelenk der Backen (7, 8) darstellen, wobei jede der Backen (7, 8) in der Verlängerung eines sekundären Griffes an dessen hinterem Ende (20a, 20b) ausgebildet ist.
- Gelenke für die hinteren Enden der Griffe (2a, 2b), jeder zusammen mit dem vorderen Ende eines der sekundären Griffe (20a, 20b).

12. Mehrzweckzange für medizinische Zwecke gemäß Anspruch 11, **gekennzeichnet dadurch,**
**dass** die Gelenke aus folgenden Elementen bestehen:
- zwei symmetrische Schalen (19a, 19b), jede mit dem vorderen Ende eines sekundären Griffes (20a, 20b) fest verbunden und jede mit einem Aufnahmehohlraum (22a, 22b) versehen, der auf einem zur Achse (26) annähernd senkrechten und zentrischen, durch die beiden Flachstellen (6, 17) gehenden Kreisbogen in die Schale (19a, 19b) eingreift, wobei diese Aufnahmehohlräume (22a, 22b) mit einer von dem sekundären Griff (20a, 20b) wegzeigenden Öffnung versehen sind;
- zwei Kerne (18a, 18b), jeder mit einem hinteren Ende eines Griffs (2a, 2b) fest verbunden und in dem Aufnahmehohlraum (22a, 22b) der Schalen (19a, 19b) angeordnet, wobei die Kerne (18a, 18b) in ihrem Aufnahmehohlraum (22a, 22b) in Translationsrichtung festgelegt sind.

13. Mehrzweckzange für medizinische Zwecke gemäß Anspruch 11, **gekennzeichnet dadurch,**
**dass** die Gelenke aus folgenden Elementen bestehen:
- zwei symmetrische Schalen (19a, 19b), jede mit dem hinteren Ende eines Griffes (2a, 2b) fest verbunden und jede mit einem Aufnahmehohlraum (22a, 22b) versehen, der auf einem zur Achse (26) annähernd senkrechten und zentrischen, durch die beiden Flachstellen (6, 17) gehenden Kreisbogen in die Schale (19a, 19b) eingreift, wobei diese Aufnahmehohlräume (22a, 22b) mit einer von dem Griff (2a, 2b) wegzeigenden Öffnung versehen sind;
- zwei Kerne (18a, 18b), jeder mit einem vorderen Ende eines sekundären Griffs (20a, 20b) fest verbunden und in dem Aufnahmehohlraum (22a, 22b) der Schalen (19a, 19b) angeordnet, wobei die Kerne (18a, 18b) in ihrem Aufnahmehohlraum (22a, 22b) in Translationsrichtung festgelegt sind.

14. Mehrzweckzange für medizinische Zwecke gemäß Anspruch 12 oder 13, **gekennzeichnet dadurch, dass**
- die Kerne (18a, 18b) kugelförmig sind,
- die Aufnahmehohlräume (22a, 22b) Hohlräume (23, 24) besitzen, deren Form und Abmessungen denen der Kerne (18a, 18b) komplementär sind.

## Claims

1. Forceps for medical use comprising two jaws (7,8) articulated on a hinge and controlled by the user from a gripping part (1), on which:
- one (7) of the jaws (7, 8) has means for pricking in the patient's tissue causing it to project towards the outside of the forceps when the jaws are closed and located at the distal end of the one of the jaws (7,8);
- one (7) of the jaws (7, 8) has two cutting blades (10) on its periphery for cutting tissue;
- at least one of the jaws (7, 8) has a cavity (13) for receiving the cut tissue,
**characterised in that**
the pricking means consist of a prick (9) causing projection towards the outside of
the forceps and towards the other jaw (8).

2. Forceps for medical use comprising two jaws (7, 8) articulated on a hinge and controlled by the user from a gripping zone (1), on which:
- one (7) of the jaws (7, 8) has means for pricking the tissue of the patient causing projection towards the outside of the forceps when the jaws are closed and located at the distal end of the one of the jaws (7, 8);
- one (7) of the jaws (7, 8) has two cutting blades (10) on its periphery for cutting tissue;
- at least one of the jaws (7, 8) has a cavity (13) for receiving the cut tissue,
**characterised in that**
the pricking means consist of two pricks (14a, 14b) each one located at the distal end of one of jaws (7, 8) and causing projection towards the outside of the forceps and towards the other jaw, two pricks (14a, 14b) being positioned side by side when jaws (7, 8) are joined together.

3. Forceps for medical use according to any of claims 1 or 2,
**characterised in that**
cutting blades (10) meet at the distal end of the jaw (7) which carries them, forming two sides of an isosceles triangle.

4. Forceps for medical use according to claim 3, **characterised in that**
blades (10) are approximately three centimetres long and the base of the triangle is approximately two centimetres long.

5. Forceps for medical use according to any of claims 1 to 4,
**characterised in that**
gripping zone (1) is formed by two branches (2a, 2b) with rings (3a, 3b) connected by a hinge (6) allowing jaws (7, 8) to pivot, each of the aforementioned jaws (7, 8) forming the extension of a branch (2a, 2b), on the side of hinge (6) opposite gripping zone (1).

6. Multipurpose forceps for medical use according to claim 5
**characterised in that**
rear part (16a, 16b) of branches (2a, 2b), located on the side of hinge (6) opposite gripping zone (1), is curved in a convex manner and is made from material elastically deformable when jaws (7, 8) are applied.

7. Multipurpose forceps for medical use according to claim 6
**characterised in that**
the curve of two rear parts (16a, 16b) is symmetrical.

8. Multipurpose forceps for medical use according to any of claims 5 to 7, **characterised in that**
forward part (15a, 15b) of branches (2a, 2b) positioned on the same side of hinge (6) as gripping zone (1) is curved in a concave manner and made of material elastically deformable when jaws (7,8) are applied.

9. Multipurpose forceps for medical use according to claim 8
**characterised in that**
the curve of the two forward parts (15a, 15b) is symmetrical.

10. Multipurpose forceps for medical use according to any of claims 5 to 9, **characterised in that**
the distance between the distal end of jaws (7, 8) and hinge (6) is three to five centimetres.

11. Multipurpose forceps for medical use according to any of claims 1
to 4 **characterised in that**
it includes:
- two branches (2a, 2b) connected by a hinge (6) and presenting a ring (3a, 3b) at their forward end so as to constitute gripping part (1);
- two secondary branches (20a, 20b) connected by a hinge (17) to allow pivoting of the jaws (7, 8), each of the aforementioned jaws (7, 8) being formed in the extension on the forward end of a secondary branch (20a, 20b);
- means for articulating the rear ends of branches (2a, 2b), each one with the forward end of one of the secondary branches (20a, 20b).

12. Multipurpose forceps for medical use according to claim 11
**characterised in that**
the articulation means include:
- two symmetrical shells (19a, 19b) each one joined to the forward end of a secondary branch (20a, 20b) and equipped each one with a hollow housing (22a, 22b) extending into shell (19a, 19b) according to a roughly perpendicular arc of a circle and centred relative to the axis (26) passing through two hinges (6, 17), the aforementioned hollow housings (22a, 22b) being equipped with an opening oriented opposite secondary branch (20a, 20b);
- two cores (18a, 18b), each one joined to the rear end of a branch (2a, 2b) and placed in hollow housing (22a, 22b) of shells (19a, 19b), traverse movement of cores (18a, 18b) being stopped in their hollow housing (22a, 22b).

13. Multipurpose forceps for medical use according to claim 11
**characterised in that**
the joining means include:
- two symmetrical shells (19a, 19b) each one joined to the rear end of a branch (2a, 2b) and equipped each one with a hollow housing (22a, 22b) extending into shell (19a, 19b) according to a roughly perpendicular arc of a circle and centred relative to axis (26) passing through two hinges (6, 17), aforementioned hollow housings (22a, 22b) being equipped with an opening oriented opposite branch (2a, 2b);
- two cores (18a, 18b) each one joined to the forward end of a secondary branch (20a, 20b) and placed in hollow housing (22a, 22b) of shells (19a, 19b), traverse movement of cores (18a, 18b) being stopped in their hollow housing (22a, 22b).

14. Multipurpose forceps for medical use according to claims 12 or
13 **characterised in that**
- cores (18a, 18b) are spherical,
- hollow housings (22a, 22b) include cavities (23, 24) have shapes and dimensions which match those of cores (18a, 18b).
